# EUROPÄISCHE PATENTSCHRIFT

(11) **EP 2 367 478 B1**
(45) Veröffentlichungstag und Bekanntmachung des Hinweises auf die Patenterteilung: **27.04.2016**
(21) Anmeldenummer: 09716604.5
(22) Anmeldetag: 27.02.2009
(51) Int. Cl.: A61B 5/06, B01F 13/08, A47J 43/046, A61B 5/07

(54) **ANORDNUNG ZUR BERÜHRUNGSLOSEN ERZEUGUNG VON DEFINIERTEN MECHANISCHEN, ELEKTRISCHEN UND MAGNETISCHEN IMPULSEN**
ARRANGEMENT FOR THE TOUCHLESS GENERATION OF DEFINED MECHANICAL, ELECTRICAL AND MAGNETIC IMPULSES
DISPOSITIF DE GÉNÉRATION SANS CONTACT D'IMPULSIONS MÉCANIQUES, ÉLECTRIQUES ET MAGNÉTIQUES DÉFINIES

(30) Priorität: 05.03.2008 DE 102008012945
(43) Veröffentlichungstag der Anmeldung: 28.09.2011
(73) Patentinhaber: Fraunhofer-Gesellschaft zur Förderung der angewandten Forschung e.V., 80686 München (DE)
(72) Erfinder: LAUSCH, Holger, 07743 Jena (DE); BRAND, Michael, 09518 Grossrückerswalde (DE); WERNER, Christoph, 07743 Jena (DE)
(74) Vertreter: Oehmke, Volker
(86) Internationale Anmeldenummer: PCT/DE2009/000284
(87) Internationale Veröffentlichungsnummer: WO 2009/109172

(56) Entgegenhaltungen:
- EP-A- 0 654 660
- EP-A1- 2 117 104
- EP-B1- 0 966 234
- WO-A-2004/057304
- US-A- 3 206 173
- US-A- 4 054 270
- US-A- 4 390 283
- US-A1- 2003 020 810
- US-A1- 2005 085 696

## Beschreibung

Die Erfindung betrifft eine Anordnung zur Erzeugung von definierten mechanischen, elektrischen und magnetischen Impulsen gemäß der Gattung der Patentansprüche. Sie ist zu Stimulationszwecken im Fitness- und Wellnessbereich ebenso verwendbar wie in der Medizin, Technik und Spielzeugindustrie.

Aus EP 0 966 234 B1 und US 6,168,780 B1 ist die Verwerdung eines im Wesentlichen kugelförmigen Markers bekannt, der sich zur magnetischen Bestimmung seiner Position in einem Organismus in einer Kapsel frei beweglich befindet. Ein kurzzeitig von außerhalb angelegtes Magnetfeld und Gegenfeld orientiert den magnetischen Marker definiert und induziert in ihm jeweils ein magnetisches Streufeld, das seiner Ortung dient. Während dieses Messzyklus darf sich der Ort des Markers nicht verändern. Ebenso soll der Marker möglichst genau und schwingungsfrei ausgerichtet und damit Trägheitseffekte als Mess- und Auswertungsfehler ausgeschlossen werden. Bekannt ist dabei auch, dem Marker eine zur Wirkstofffreisetzung günstige Oberflächenstruktur und eine Ablage seines Masseschwerpunktes von seinem geometrischen Schwerpunkt zu erteilen.
Bekannt ist aus WO 2007/131503 A2 eine Anordnung, in der ein um seine Symmetrieachse rotierender, diametral magnetisierter Primär-Permanentmagnet einen dreiachsig frei gelagerten, diametral magnetisierten Sekundär-Permanentmagneten in Drehung versetzt. Mit dieser Anordnung kann das rotierende Streufeld des Sekundärmagneten direkt und ohne Einfluss des ständig rotierenden Primärmagneten gemessen werden, weil der Einfluss seines Magnetfeldes durch eine Kompensationseinrichtung ausgeschaltet ist. Auch bei dieser Anordnung treten mehr oder weniger große, ungewollte und unkontrollierbare Eigenschwingungen auf, die das Messergebnis nachteilig beeinflussen.
Ebenfalls bekannt sind Magnetrührer, siehe bspw. EP 0 654 660 B1 oder US 3,206,173 A, welche in chemischen Labors zum Rühren von Flüssigkeiten verwendet werden. Die Flüssigkeit befindet sich in einem Becherglas, das über einem mit regelbarer Geschwindigkeit rotierenden äußeren Magneten positioniert ist. Durch den äußeren, meist stabförmigen Magneten wird ein in der Flüssigkeit befindlicher, vorzugsweise stabförmiger und zum äußeren Magneten im Wesentlichen parallel gerichteter Magnet in gleichlaufende Drehungen versetzt. Mit dem stabförmigen Magnetrührer wird in der Flüssigkeit eine durchmischende Strömungsdynamik erzielt, die mit einem kugelförmigen Rührer keinesfalls erreichbar ist. Auch treten ungewollte Vibrationseffekte auf die eine optimale mischende Strömung beeinträchtigen. Diese werde gegebenenfalls mit einem Ausgleichskörper beseitigt.
In WO 2004/057304 A1 stellt der Sekundärmagnet ebenfalls einen Magnetrührer dar, welcher allerdings in einer miniaturisierten Ausführung gestaltet ist. Der Sekundärmagnet (Rührkörper) kann zur Homogenisierung von kleinen Flüssigkeitsmengen z. B. in Injektionsspritzen eingesetzt werden. In der Beschreibung der o. g. WO-Schrift werden auch Ausführungen genannt, in welchen auch mehrere Rührkörper zum Einsatz kommen. Die Rührkörper sind hier kugelförmig bis elliptisch ausgeführt. In der EP 0 654 600 A1 wird beschrieben, dass der Sekundärmagnet ebenfalls als Rührkörper ausgestaltet ist. Es ist eine Viskositätsmessung über die Detektion des Nachlaufes des Sekundärmagneten integriert.
In der US 2005/0856696 A1 ist der Sekundärmagnet als einzelner Körper mit der Kapsel fest verbunden. Der Sekundärmagnet ist in seiner Kapsel nicht beweglich angeordnet. Die dann durch den Sekundärmagneten mittels des Primärmagneten bewegte Kapsel, dient zur Positionierung des Fokus eines integrierten optio-sensorischen Systems.
Die EP 2 117 104 A1 beschreibt zwei oder mehrere relativ zueinander rotierbare Magneten in einem Gehäuse. Diese Magnete sind Permanentmagnete, welche in einer Kaskade angeordnet sind. Der Bewegungsfluss in der Kaskade wird durch die Anziehungs- und Abstoßungskräfte der Permanentmagnete gewährleistet. Alle Magnete sind in parallelen Ebenen ihrer Magnetisierungsrichtung angeordnet. In Einzelfällen ist einer der Magnete nur soweit gekippt, dass die Funktion der Kaskade noch gewährleistet ist.
Keine der in EP 2 117 104 A1 dargestellten Anordnungen dient der einfachen Erzeugung mechanischer, elektrischer oder magnetischer Impulse. Es werden lediglich unterschiedliche Permanentmagnetanordnungen zur Bewegungssteuerung eines Instrumentes oder Sensors unter zwingender Zuhilfenahme von mechanischen Führungen beschrieben, die zudem alle in einem Gehäuse integriert sind.
In US 2003/0020810 A1 wird eine Kapsel mit integrierten optio-sensorischen System beschrieben. Das System wird durch eine integrierte Batterie betrieben und ist somit nicht für den Langzeitbetrieb geeignet. Durch ein äußeres bewegtes Magnetfeld kann, z. B. die Kapsel, durch die Lumina eines menschlichen Organismus transportiert werden. Zur Induzierung einer gleichmäßigen, näherungsweisen uniaxialen rotierenden Bewegung der Kapsel, ist diese an der Außenseite mit mechanischen Elementen versehen. Das in US 4,390,283 A beschriebene Magnetrührer-Arrangement ist so gestaltet, dass ein Rührkörper, welcher sich innerhalb des zu mischenden Mediums befindet, durch äußere starr angeordnete promäre Permanentmagneten festgehalten wird. Der Rühreffekt kommt durch die rotierende Bewegung des Gefäßes mit dem Medium zu Stande.
Die Veröffentlichung US 4,054,270 A beschreibt einen Magnetrührer zur Vermischung zweier Stoffkomponenten. Der Sekundärmagnet ist wie bei allen Magnetrührern zwecks chemischer Indifferenz zum Medium gekapselt. Der mit der Rührkörperkapsel fest verbundene Sekundärmagnet rotiert orthogonal zur Rotationsrichtung des Primärmagneten.

Durch die Erfindung soll eine einfach gestaltete und wirkungsvolle Anordnung zur berührungslosen Erzeugung von definierten mechanischen, elektrischen und magnetischen Impulsen unter Anwendung magnetischer Prinzipien geschaffen werden. Durch die definierte Lagerung mindestens eines sich an unzugänglichen Stellen befindlichen Sekundärmagneten soll dieser oder seine Lagerungsstruktur zu definierten Bewegungen angeregt werden, die an oder in einem Objekt bzw. Körper definierte Bewegungs- und Feldmuster erzeugen, die direkt oder indirekt appliziert, bspw. in der Medizin biokompatibel zu therapeutischen Zwecken verwendet werden können. Gemäß der Erfindung werden die aufgezeigten Mängel des Standes der Technik durch die kennzeichnenden Merkmale des ersten Patentanspruchs behoben und die erfindungsgemäße Lösung durch die Merkmale der Unteransprüche weiter ausgestaltet.
Der einem oder beiden Magneten zugeordnete Aktor kann mechanischer oder magnetischer Art sein. Mechanischer Art ist es, wenn bspw. die magnetischen Schwerpunkte nicht auf den Rotationsachsen der Primärmagneten und/oder Sekundärmagneten liegen. Dabei kann den Primär- und/oder den Sekundärmagneten zur Vermeidung von mechanischen Unwuchten jeweils ein magnetischer, magnetisierbarer oder unmagnetischer Ausgleichkörper zugeordnet sein.
Ebenfalls mechanischer Art ist der Aktor bspw., wenn der Sekundärmagnet in einer Kapsel allseitig frei rotieren oder sich definiert bewegen kann, deren Durchmesser deutlich größer ist als der Durchmesser des kugelförmigen Sekundärmagneten. Zwischen Sekundärmagneten und Kapsel kann ein schwachviskoses Medium vorgesehen sein, das die allseitig freie Drehung des Sekundärmagneten gewährleistet.
Bei den magnetischen Aktoren handelt es sich um Permanentmagnete oder Elektromagnete, die vorzugsweise dem Sekundärmagneten zugeordnet sind und deren magnetische Wirkung regelmäßig deutlich geringer ist als die der Primär- oder Sekundärmagnete. Die Elektromagnete können als Spulen gestaltet sein, die in der Umgebung des Sekundärmagneten angeordnet sind und denen Mittel zur vorzugsweise induktiven Stromversorgung unmittelbar zugeordnet sind.
Die mechanischen und die magnetischen Aktoren können miteinander kombiniert verwendet werden.
Sowohl der Sekundärmagnet als auch die magnetischen Aktoren und ggf. die Mittel zur Stromversorgung sind in einem Gehäuse untergebracht, das die Größe von Arzneikapseln haben kann. Jeder Sekundärmagnet bzw. die äußere der ihn umgebenden Kapseln ist im Gehäuse mit Hilfe einer Haltestruktur angeordnet.
Die Zuleitung der Impulse des Sekundärmagnets zu einem Objekt oder Körperteil kann beliebig und entsprechend dem Verwendungszweck gestaltet sein. Befindet sich der Sekundärmagnet in einem Gehäuse, so kann die Ableitung der Impulse flächenhaft über die Gehäusewand oder punktuell über Noppen oder einzelne Kontaktkörper in günstiger Weise geschehen. Die in den Mitteln zur induktiven Stromversorgung erregten Spannungen können nach ihrem Durchgang durch mindestens eine Steuer- und Kommunikationseinheit vorteilhaft als elektrische Impulse (Reizströme mit beliebigen Verläufen - Gleich- bzw. Wechselströme, Modulation, Frequenzmuster) an punktförmigen Einzelelektroden oder an Flächenelektroden abgegriffen werden. Am Gehäuse können die Flächenelektroden als Kappen- oder Mantelelektroden gestaltet sein. Ebenso wie die Anzahl der einander korrespondierenden Primär- und Sekundärmagnete an sich beliebig sein kann, so können auch ihre Formen grundsätzlich beliebig sein. Als anwendungsseitig und konstruktiv vorteilhaft hat sich jedoch die zylindrische Gestaltung des Primärmagneten und die kugelförmige Gestaltung des Sekundärmagneten, beide Gestaltungen verbunden mit einer diametralen N-, S-Magnetisierung, ergeben.
Die Erfindung wird nachstehend an Hand von vierzehn Ausführungsbeispielen näher beschrieben, die in der Zeichnung schematisch dargestellt sind und weitere Merkmale der Erfindung enthalten. Es zeigen:
- Fig. 1: eine dem Stand der Technik entsprechende Magnetanordnung im Grundriss a und Aufriss b,
- Fig. 2: eine erfindungsgemäße Magnetanordnung im Grundriss a und Aufriss b,
- Fig. 3: eine von Fig. 2 abweichende Gestaltung der Anordnung des Sekundärmagneten im Grundriss,
- Fig. 4: die Grundrissdarstellung einer weiteren, von Fig. 2 abweichende Gestaltung der Anordnung des Sekundärmagneten,
- Fig. 5: die Grundrissdarstellung einer dritten Gestaltungsmöglichkeit für die Anordnung des Sekundärmagneten,
- Fig. 6: den Grundriss einer Lagerungsmöglichkeit für den Sekundärmagneten,
- Fig. 7: eine zweite Lagerungsmöglichkeit des Sekundärmagneten im Grundriss,
- Fig. 8: die Grundrissdarstellung einer dritten Lagerungsmöglichkeit für den Sekundärmagneten,
- Fig.9: eine Zuordnung von permanentmagnetischen Mitteln zur Magnetfeldbeeinflussung zum Sekundärmagneten im Grundriss,
- Fig. 10: eine Grundrissdarstellung der Zuordnung von elektromagnetischen Mitteln zur Magnetfeldbeeinflussung zum Sekundärmagneten,
- Fig. 11: eine von Fig. 10 abweichende Zuordnung der elektromagnetischen Mittel zum Sekundärmagneten zur Magnetfeldbeeinflussung,
- Fig. 12: eine Kopplung des Sekundärmagneten mit einer induktiven Spulenanordnung zur Reizstromerzeugung,
- Fig. 13: eine Kopplung des Sekundärmagneten mit einer induktiven Spulenanordnung zur Reizstromerzeugung mit gegenüber der Fig. 12 veränderten Stromabgriff,
- Fig. 14: eine Kopplung des Sekundärmagneten mit einer induktiven Spulenanordnung zur Reizstromerzeugung mit einer weiteren Variante des Stromabgriffs und
- Fig. 15: ein System von Sekundärmagneten zur Ausrichtung am Magnetfeld des Primärmagneten.

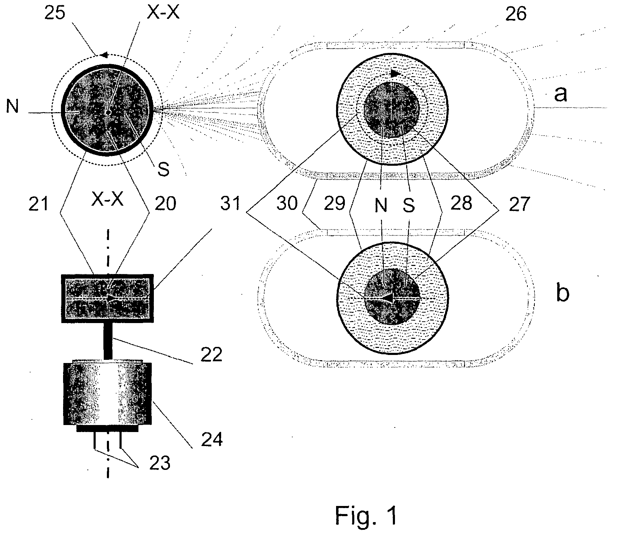

In Fig. 1 ist ein als Zylinder ausgebildeter Primärmagnet 20 mit Polen N, S dargestellt, der mit einem Träger 21 starr verbunden und mit diesem um seine geometrische Achse X-X drehbar ist. Der Träger 21 sitzt auf einer Antriebswelle 22 eines mit Anschlüssen 23 versehenen Elektromotors 24, der im bestromten Zustand den Primärmagneten 20 in Richtung eines Pfeils 25 um die Achse X-X dreht. Auf der Drehachse X-X befinden sich der mechanische und der magnetische Schwerpunkt des Permanentmagneten 20. Dieser generiert ein relativ starkes Magnetfeld, das durch Feldlinien 26 angedeutet ist.
Zu der in Fig. 1 dargestellten Magnetanordnung gehört ein im vorliegenden Fall als Kugel gestalteter Sekundärmagnet (aus permanentmagnetmagnetischem oder magnetisierbarem Material) 27, der ebenfalls zwei Pole N, S besitzt, ein Magnetfeld geringerer Stärke generiert und in einem gering-viskosen Medium 28 in einer Kapsel 29 allseitig beweglich gelagert ist, welche von einem Gehäuse 30 umgeben ist. Dreht sich der Primärmagnet 20 in Richtung des Pfeils 25, so erfährt der Sekundärmagnet 27 eine gegenläufige Drehung in Richtung eines Pfeils 31, weil er sich im Magnetfeld des Primärmagneten 20 befindet. Dabei treten kinetische Effekte zwischen den beiden Magneten auf, die generell abhängig sind
- von den magnetischen Momenten des/der Primär- und Sekundärmagneten,
- vom Abstand zwischen Primär- und Sekundärmagneten,
- von der Viskosität des Lagermediums des Sekundärmagneten,
- vom Verhältnis des massebehafteten Drehmoments des Sekundärmagneten zum Gravitationsfeld,
- von den Lageverhältnissen zwischen geometrischem Schwerpunkt, Masseschwerpunkt und magnetischem Schwerpunkt des Primärkörpers (Primärmagnet und Träger) zur Erzeugung definierter Magnetfeldformen,
- vom Verhältnis der Massen und/oder Dichten des Sekundärmagneten und des gesamten Innenkörpers (Sekundärmagnet und Kapsel und Lagermedium),
- von den Lageverhältnissen zwischen geometrischem Schwerpunkt, Masseschwerpunkt und magnetischem Schwerpunkt des Sekundärkörpers,
- vom Bewegungsfreiraum des Sekundärmagneten in der Kapsel,
- vom Verhältnis von Roll- und Gleitreibung des Sekundärmagneten und des Lagermediums an der Kapsel,
- von der Formengleichheit des Sekundärmagneten und der umgebenden Kapsel,
- von der Positionierung der Kapsel mit dem rotierenden Sekundärmagneten bezüglich des geometrischen Schwerpunktes des Gehäuses bzw. des gesamten Sekundärsystems,
- vom einheitlichen oder räumlich unterschiedlichen Dämpfungsgrad der Lagerung der Kapsel im umgebenden Gehäuse bzw. gesamten Sekundärsystem sowie
- von einer weiteren, vorzugsweise magnetisch wirksamen Beeinflussung des primärmagnetischen Feldes im oder am Sekundärsystem.

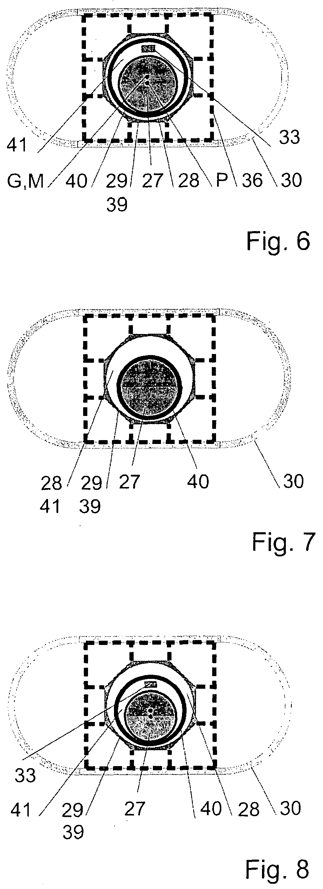

Je größer die magnetischen Momente von Primär- und Sekundärmagnet 20, 27 sind, desto größer bzw. weitreichender ist die Kraftübertragung. Bedingt durch den Motorantrieb 23, 22 ist die Bewegung des Primärmagneten 20 gravitationsunabhängig. Das magnetische Moment des Sekundärmagneten 27 muss wegen seiner freien Beweglichkeit bzw. allseitigen Drehbarkeit und dem damit einhergehenden Einfluss der Gravitation im optimalen Verhältnis zu seiner Masse gewählt werden. Das magnetische Moment des Primärmagneten 20 sowie das Verhältnis des magnetischen Moments des Sekundärmagneten 27 oder eines den Sekundärmagneten enthaltenden Sekundärkörpers (siehe die Figuren 6, 7, 8) zu seiner Masse bestimmen den maximal möglichen Abstand zwischen Primär- und Sekundärmagnet. Je höher die Viskosität des Lagermediums 28 ist, desto größer muss die Kraftkupplung zwischen Primär- und Sekundärmagnet sein. Deshalb soll das Medium 28 eine möglichst geringe Viskosität aufweisen oder gasförmig sein. Unter Berücksichtigung der oben aufgelisteten Abhängigkeiten werden in den nachfolgenden Ausführungsbeispielen diejenigen Varianten realisiert, die am besten zur berührungslosen Erzeugung von definierten mechanischen, elektrischen und magnetischen Impulsen, bspw. Vibrationen und Reizströmen geeignet erscheinen. Dabei geht es insbesondere um die Gestaltung der primären und sekundären Rotationskörper und die gegenseitigen Lagen von ihren geometrischen Schwerpunkten, Masseschwerpunkten und magnetischen Schwerpunkten.

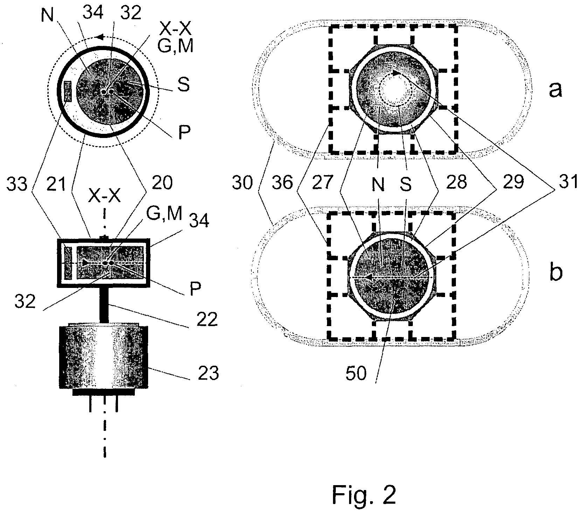

In Fig. 2 treibt (wie in Fig. 1) ein Motor 23 über eine Welle 22 einen Träger 21 zu Drehungen um eine Rotationsachse X-X an. Der Träger 21 umschließt einen zylindrischen, diametral zu einer Axialebene 32 mit Polen N, S versehenen Primärmagneten 20 sowie einen vorzugsweise magnetisch neutralen Ausgleichkörper 33, die beide starr mit dem Träger 21 verbunden sind und mit diesem einen primären Rotationskörper 34 bilden. Die Anordnung ist so getroffen, dass die geometrische Achse des Rotationskörpers 34 mit der Drehachse X-X koinzidiert, dass der Geometrieund der Masseschwerpunkt des Rotationskörpers G und M auf der Achse X-X und ineinander liegen, dass aber der Magnetschwerpunkt P neben der Achse X-X liegt, und zwar in Fig. 2 rechts daneben. Dabei verhindert der Ausgleichskörper 33 mechanische Unwuchten beim Drehen des Rotationskörpers 34. Die Ablage des Magnetschwerpunktes P vom Geometrie- und Masseschwerpunkt G, M bewirkt eine periodische Verlagerung des Magnetfeldes des Primärmagneten 20.
Im Magnetfeld des Primärmagneten 20 ist ein Gehäuse 30 mit einer Haltestruktur 36 für eine Kapsel 29 vorgesehen, die einen Sekundärmagneten 27 enthält. Die Kapsel 29 und der Sekundärmagnet 27 sind zentral im Gehäuse 30 angeordnet. Der Sekundärmagnet 27 ist in der Kapsel 29 in einem Medium 28 geringer Viskosität allseitig drehbar gelagert; seine freie Beweglichkeit ist dadurch eingeschränkt, dass der kugelförmige Innenraum der Kapsel 29 nur wenig größer ist als der kugelförmige Sekundärmagnet 27.
Beim Drehen des Primärmagneten 20 um die Achse X-X werden also definierte Magnetfeldformen (modulierte Drehfelder) erzeugt, die dem Sekundärmagneten 27 zusätzlich zur Rotation in Richtung eines Pfeils 31 eine translatorische Bewegung zur Erzeugung linearer oder radialer Vibrationsmuster erteilen. Die Vibrationsfrequenz ist dabei an die Rotationsfrequenz des Primärmagneten 20 gebunden. Zum Generieren definierter Feldformen kann der Ausgleichkörper 33 auch aus magnetischen bzw. magnetisierbaren Materialien bestehen. Unwuchten können auch durch die Verwendung von Materialien gleicher Dichte zur Herstellung des Trägers 21 und des Primärmagneten 20 (einschließlich der Füllung zwischen beiden) verwendet werden. Schließlich ist es möglich, zur Vermeidung von Unwuchten die Dichten im Träger 21 und/oder im Füllmaterial entsprechend zu gestalten.

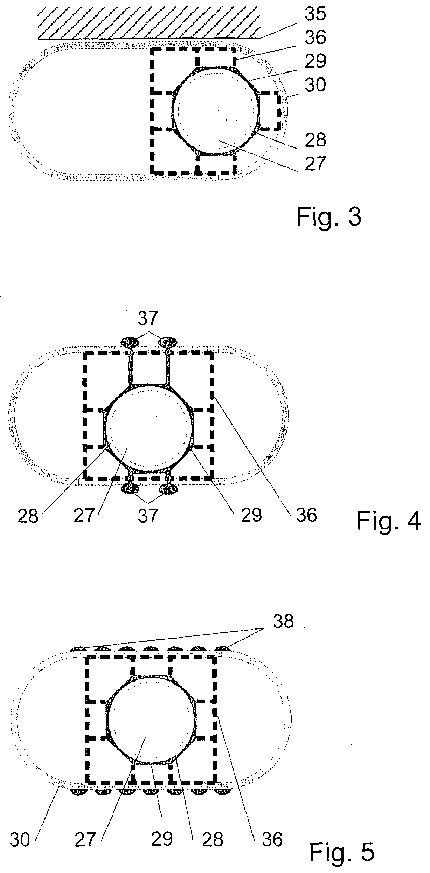

Die folgenden Figuren 3, 4, 5 beinhalten unterschiedliche Anordnungen der Kapsel 29 im Gehäuse 30 sowie der Herausführung der Vibrationen aus dem Gehäuse. Der Sekundärmagnet 27 ist wie zu Fig. 1 oder 2 beschrieben gestaltet. In Abhängigkeit vom Abstand der beiden Magnete 20 und 27, vor allem im Nahbereich, wird ihre Rotation von einer translatorischen Bewegung überlagert, die durch ihre magnetische Anziehungskraft bedingt ist. Diese Tatsache kann bei der Gestaltung der erfindungsgemäßen Anordnung, insbesondere bei der Gestaltung der Sekundäreinheit 27, 29 berücksichtigt werden. Wie in Fig. 2 ist der Sekundärmagnet 27 in einem Medium 28 in der Kapsel 29 gelagert, deren Innenraum nur einen wenig größeren Durchmesser aufweist als der Sekundärmagnet 27.
In Fig. 3 ist der Sekundärmagnet 27 mit der umgebenden Kapsel 29 mit Hilfe der Haltestruktur 36 exzentrisch im Gehäuse 30 gelagert. Ihre Vibrationen geben sie über die zylindrische Außenfläche des Gehäuses 30 an ein andeutungsweise dargestelltes Objekt 35 ab. Die exzentrische Lage der Sekundäreinheit 27, 29 erzeugt Bewegungsmuster bzw. zusätzliche Bewegungsmuster die zur Beeinflussung der Vibration führen.
In Fig. 4 sind der Sekundärmagnet 27 und die ihn umgebende Kapsel 29 ebenfalls exzentrisch im Gehäuse 30 angeordnet. Ihre Vibration wird über Teile der Haltestruktur 36 nach außen geführt und ist an mechanischen Kontaktkörpern 37 abnehmbar.
Fig. 5 zeigt das Gehäuse 30, in dem die Kapsel 29 mit dem Sekundärmagneten 27 wie in Fig. 2 zentrisch angeordnet ist, bei dem die Vibrationen von Sekundärmagnet 27 und Kapsel 29 über an der Gehäuseaußenfläche angebrachte Noppen 38 an ein nicht dargestelltes, das Gehäuse 30 zumindest teilweise umgebendes Körperteil oder Objekt abgegeben werden.

In Fig. 6 ist im Gehäuse 30 mit Hilfe der Haltestruktur 36 eine Außenkapsel 39 befestigt, in der eine Innenkapsel 40 von nur wenig geringerem Durchmesser als die Außenkapsel 39 in einem Medium 28 befindlich gelagert ist. In der Innenkapsel 40 sind der Sekundärmagnet 27 und ein Ausgleichkörper 33 mit Hilfe eines Füllmediums 41 fest so angebracht, dass der magnetische Schwerpunkt P nicht mit dem geometrischen Schwerpunkt G und dem Masseschwerpunkt M zusammenfällt. Hingegen koinzidiert der Masseschwerpunkt M mit dem geometrischen Schwerpunkt G. Die Anordnung ist also ähnlich der des primären Rotationskörpers 34 in Fig. 2. In diesem Fall wird das Magnetfeld des Sekundärmagneten 27 bewegt und verändert, was in gleicher Weise zu gewünschten Vibrationen führt.
Es ist auch möglich, in einer Anordnung die Innenkapselgestaltung der Fig. 6 mit dem primären Rotationskörper 34 der Fig. 2 zu kombinieren, um ein gewünschtes Vibrationsmuster zu erhalten.

In den Figuren 7 und 8 ist der Sekundärmagnet 27 bzw. die Innenkapsel 40 von deutlich geringerem Durchmesser als die Kapsel 29 bzw. die Außenkapsel 39, die im übrigen mit einem Medium 28 gefüllt und im Gehäuse 30 mit Hilfe der Haltestruktur 36 angebracht ist. Es treten bei bestimmten Drehfrequenzen der Magnete 20, 27 resonanzbedingte Vibrationen auf, die von den in den Kapseln 29, 39 auftretenden Reibungseffekten und damit vom Medium 28 und der Beschaffenheit der an das Medium 28 angrenzenden Magnet- und Kapselflächen abhängen. Diese Vibrationen überlagern sich mit den aus der exzentrischen Lagerung des Sekundärmagneten 27 in der Kapsel 29 bzw. der Innenkapsel 40 in der Außenkapsel 39 herrührenden Vibrationen zu gewünschten und nach außen ableitbaren Vibrationsbildern.

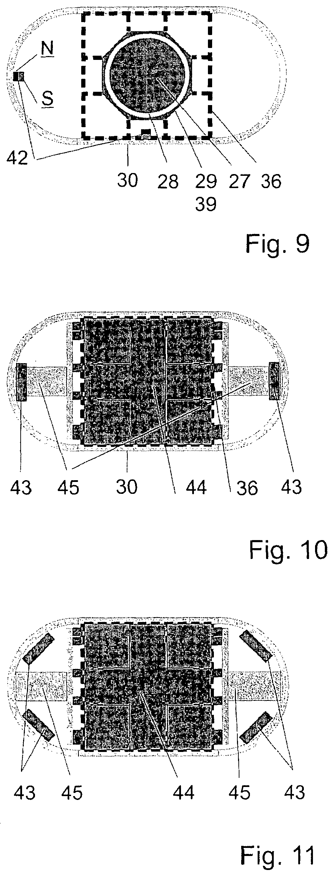

In Fig. 9 ist im Gehäuse 30 mittels der Haltestruktur 36 die Kapsel 29 befestigt, in welcher der sphärische Sekundärmagnet 27 in einem Medium 28 allseitig drehbar gelagert ist. An der Innenwand des Gehäuses 30 sind zwei Permanentmagnete 42 befestigt, die auf den Sekundärmagneten 27 ablenkend und auf das resultierende Magnetfeld von Primär- und Sekundärmagnet deformierend derart einwirken, dass der rotierende Sekundärmagnet 27 in Schwingungen gerät, die mit Mitteln, wie in den Figuren 3, 4, 5 dargestellt, nach außen abgeleitet werden können. Es wird also die mechanische Beeinflussung des resultierenden Magnetfeldes des Primär- und des Sekundärmagneten im vorliegenden Ausführungsbeispiel wie in den folgenden Ausführungsbeispielen der Figuren 10 und 11 durch eine originär magnetische Beeinflussung ersetzt. Diese Beeinflussung kann sowohl mit Permanentmagneten als auch mit Elektromagneten erfolgen.

In Fig. 10 sind in einem Gehäuse 30 zwei elektrische Spulen 43 (mit oder ohne Kern) an den Kappen angeordnet. Im mittleren Teil des Gehäuses 30 befindet sich die Haltestruktur 36 mit der Kapsel 29 und dem Sekundärmagneten 27 (ähnlich der Fig. 9). Letztere sind durch Mittel zur induktiven Stromerzeugung 44 für die Spulen 43 verdeckt, wie sie bspw. in der WO2007/131503 beschrieben sind, auf die hiermit ausdrücklich Bezug genommen wird, und mit denen der Sekundärmagnet 27 zusammenwirkt. Die Stromzuführungs- und Ansteuermittel zu den Spulen 43 sind mit 45 bezeichnet. Die Spulen 43 erzeugen geeignete, gerichtete Magnetfelder, welche die Bewegung des Sekundärmagneten 27 einschließlich seiner Kapsel 29 beeinflussen. Durch unterschiedliche Ansteuerung der einzelnen Spulen 43 können unterschiedliche, jedoch definierte Vibrationsmuster am Sekundärmagneten 27 erzeugt und vom Gehäuse 30 an seine Umgebung abgegeben werden.

Abweichend von Fig. 10 sind in Fig. 11 vier Spulen 43 zur gesteuerten Vibrationserzeugung vorgesehen. Im übrigen gilt das zu Fig. 10 Gesagte zumindest sinngemäß. Durch die Verwendung von vier Spulen werden die Möglichkeiten zur Erzeugung von auf den Sekundärmagneten wirkenden Kräften und damit von Vibrationsmustern und deren Anwendungsbereich erweitert.

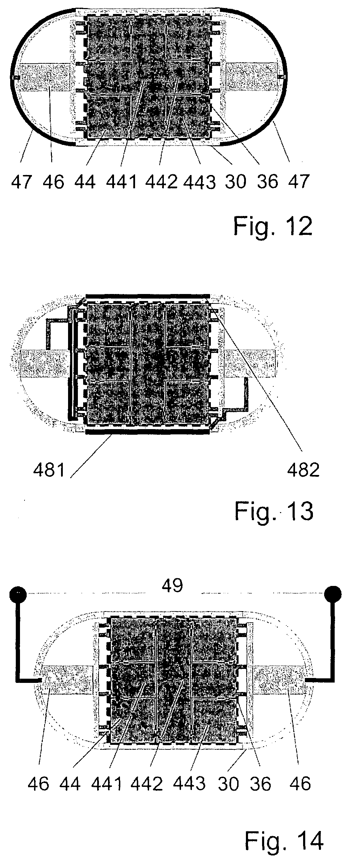

In Fig. 12 sind ähnlich wie in Fig. 11 in einem Gehäuse 30 Mittel zur induktiven Stromerzeugung 44 vorgesehen, die einen Sekundärmagneten 27 mit seiner Lagerungskapsel und wesentliche Teile einer Haltestruktur 36 verdecken. Beim Drehen des Sekundärmagneten werden in den Induktionsspulen 441, 442, 443 der Stromerzeugungsmittel 44 Ströme erzeugt, die von der Lage des Sekundärmagneten 27 bezüglich der Induktionsspulen 441, 442, 443 abhängen. Die so in der Spulenanordnung 441, 442, 443 induzierten Spannungen können direkt oder über digitale Signalgeneratoren in einer Steuer- und ggf. Kommunikationseinheit 46 zu an den Gehäusekappen angebrachten Elektroden 47 geführt werden. Dort liegen sie bspw. zur definierten, von außen berührungslosen, biokompatiblen und lokalen Erzeugung von Reizströmen an, die therapeutische Verwendung finden können.

Fig. 13 unterscheidet sich von Fig. 12 dadurch, dass anstatt der Kappenelektroden Mantelelektroden 481 und 482 verwendet werden.

In Fig. 14 werden die in der Spulenanordnung 441, 442, 443 induzierten Spannungen über eine Steuereinheit 46 Anschlusskontakten 49 außerhalb des Gehäuses 30 zum Abgriff zugeführt.

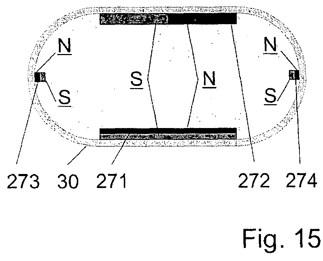

In Fig. 15 fallen Haltestruktur und Gehäuse zusammen. Demzufolge sind die dargestellten Magnete 271 bis 274 Sekundärmagnete in Kombination mit den Polungen N, S und fest mit dem Gehäuse 30 verbunden. Jeder der Sekundärmagnete kann für sich in dem Gehäuse angebracht und wirksam sein. Die Ausrichtung des durch die Sekundärmagnete erzeugten Magnetfeldes am Primärmagnetfeld bewirkt definierte Bewegungsmuster des Gehäuses 30.

Die Erfindung erschöpft sich nicht in den vorstehenden Ausführungsbeispielen. Sie ist weder an eine bestimmte Anzahl noch an eine bestimmte Form der Primär- und Sekundärmagnete gebunden. Die zu einzelnen Ausführungsbeispielen genannten Merkmale können fachgerecht in andere Ausführungsbeispiele eingeführt werden. Auch sind unterschiedliche Kombinationen von vorstehend genannten Merkmalen zu neuen Ausführungsformen möglich. Überhaupt können alle in dieser Beschreibung und den Ansprüchen genannten sowie in der Zeichnung dargestellten Merkmale sowohl einzeln als auch in beliebiger Kombination miteinander erfindungswesentlich sein. Es muss nur gewährleistet sein, dass der/die Sekundärmagnet/e sich in einer Lagerungsstruktur befinden, die in einem resultierenden definierten Magnetfeld von Primärmagneten, Sekundärmagneten und ggf. Zusatzmagneten mechanische, elektrische und magnetische Impulsgebungen zulassen.

### Bezugszeichenliste

- 20: Primärmagnet
- 21: Träger
- 22: Antriebswelle
- 23: Anschlüsse
- 24: Elektromotor
- 25, 31: Pfeile
- 26: Feldlinien
- 27, 271, 272, 273, 274: Sekundärmagnet
- 28: Medium
- 29: Kapsel
- 30: Gehäuse
- 32: Axialebene
- 33: Ausgleichskörper
- 34: Rotationskörper
- 35: Objekt, Körperteil
- 36: Haltestruktur
- 37: Kontaktkörper
- 38: Noppen
- 39: Außenkapsel
- 40: Innenkapsel
- 41: Füllmedium
- 42: Permanentmagnete
- 43: Spulen
- 44: Mittel zur induktiven Stromerzeugung
- 45: Stromzuführungs- und Ansteuermittel
- 46: Steuer- und Kommunikationseinheit
- 47: (Kappen-)Elektroden
- 481, 482: Mantelelektroden
- 49: Anschlusskontakte
- 50: Durchmesserebene
- 441, 442, 443: Induktionsspulen
- X-X: Achse
- N, S: Pole
- G: Geometrieschwerpunkt
- M: Masseschwerpunkt
- P: Magnetschwerpunkt

## Patentansprüche

1. Anordnung zur berührungslosen Erzeugung von definierten mechanischen, elektrischen und magnetischen Impulsen mit mindestens einem durch einen ersten Aktor (21, 22, 23, 24) bewegbaren Primärmagneten (20), in dessen Magnetfeld (26) sich mindestens ein in einem Gehäuse (30) vom Primärmagneten (20) bewegbarer Sekundärmagnet (27) befindet, wobei
- im Gehäuse (30) eine Lagerungsstruktur (28, 29, 36) vorhanden ist, in der der Sekundärmagnet (27) angeordnet ist,
- dem Sekundärmagneten (27) mindestens ein magnetischer Aktor (42, 43) zugeordnet ist, der auf den Sekundärmagneten (27) ablenkend und auf ein resultierendes Magnetfeld von Primärmagneten (20) und Sekundärmagneten (27) einwirkt, so dass Bewegungsimpulse des Sekundärmagneten (27) angeregt werden.

2. Anordnung gemäß Anspruch 1, **dadurch gekennzeichnet, dass** der Sekundärmagnet (27) mittels eines Mediums geringer Viskosität (28) in einer Kapsel (29) allseitig beweglich gelagert ist, deren Innenabmessungen erkennbar größer sind als die Abmessungen des Sekundärmagneten (27).

3. Anordnung gemäß Anspruch 1, **dadurch gekennzeichnet, dass** der Sekundärmagnet (27) in einer Innenkapsel (40) angeordnet ist, die sich in einer Außenkapsel (39) befindet, deren Abmaße geringfügig größer sind, als die Abmaße der Innenkapsel (40).

4. Anordnung gemäß Anspruch 1, **dadurch gekennzeichnet, dass** der Sekundärmagnet (27) in einer Innenkapsel (40) angeordnet ist, die sich in einer Außenkapsel (39) befindet, deren Abmaße deutlich größer sind, als die Abmaße der Innenkapsel (40).

5. Anordnung gemäß Anspruch 3 oder 4, **dadurch gekennzeichnet, dass** dem Sekundärmagneten (27) mindestens ein Ausgleichkörper (33) zur Vermeidung von mechanischen Unwuchten zugeordnet ist.

6. Anordnung gemäß Anspruch 5, **dadurch gekennzeichnet, dass** der Ausgleichkörper (33) aus einem magnetischen oder magnetisierbaren Material besteht.

7. Anordnung gemäß Anspruch 1, **dadurch gekennzeichnet, dass** Übertragungsmittel (37) vorgesehen sind, welche die Bewegungsimpulse des Sekundärmagneten (27) über die Lagerungsstruktur (28, 29, 36) aus dem Gehäuse (30) nach außen übertragen.

8. Anordnung gemäß Anspruch 7, **dadurch gekennzeichnet, dass** der mindestens eine magnetische Aktor (42, 43) als zwei Permanentmagnete (42) ausgebildet ist.

9. Anordnung gemäß Anspruch 1, **dadurch gekennzeichnet, dass** der mindestens eine magnetische Aktor (42, 43) als zwei stromdurchflossene Spulen (43) ausgebildet ist.

10. Anordnung gemäß Anspruch 1 oder 7, **dadurch gekennzeichnet, dass** im Gehäuse (30) Energiequellen vorhanden sind.

11. Anordnung gemäß Ansprüchen 9 und 10, **dadurch gekennzeichnet, dass** die Energiequellen Mittel zur induktiven Stromerzeugung (44) für die stromdurchflossenen Spulen (43) sind.

12. Anordnung gemäß Anspruch 11, **dadurch gekennzeichnet, dass** dem Sekundärmagneten (27) als Aktor das Mittel zur induktiven Stromerzeugung (44) zugeordnet ist.

13. Anordnung gemäß Anspruch 12, **dadurch gekennzeichnet, dass** die durch die Mittel zur induktiven Stromerzeugung (44) erregten Spannungen direkt oder über eine Steuer- und Kommunikationseinheit (46) als elektrische Impulse mittels Elektroden applizierbar sind.

14. Anordnung gemäß Anspruch 10 und 13, **dadurch gekennzeichnet, dass** am Gehäuse (30) außen Elektroden (47, 481, 482, 49) zur Abgabe der elektrischen Impulse vorgesehen sind.

15. Anordnung gemäß Anspruch 1 und 7, **dadurch gekennzeichnet, dass** die Lagerungsstruktur (28, 29, 36) mit dem Gehäuse (30) eins ist.

16. Anordnung gemäß Anspruch 15, **dadurch gekennzeichnet, dass** die Sekundärmagnete (271, 272, 273, 274) mit dem Gehäuse (30) fest verbunden sind.

## Claims

1. Arrangement for the contact-less generation of defined mechanical, electrical and magnetic impulses having at least one primary magnet (20) which can be moved by a first actuator (21, 22, 23, 24), at least one secondary magnet (27), which can be moved by the primary magnet (20) in a housing (30), being located in the magnetic field (26) of the primary magnet (20), wherein
- a supporting structure (28, 29, 36) is present in the housing (30), with the secondary magnet (27) being arranged in the supporting structure (28, 29, 36),
- at least one magnetic actuator (42, 43) is assigned to the secondary magnet (27), which magnetic actuator (42, 43) has a deflective effect on the secondary magnet (27) and influences a resulting magnetic field of primary magnets (20) and secondary magnets (27), so that movement impulses of the secondary magnet (27) are triggered.

2. Arrangement according to claim 1, **characterized in that** the secondary magnet (27) is mounted so as to be movable on all sides by means of a low-viscosity medium (28) in a capsule (29) whose internal dimensions are visibly larger than the dimensions of the secondary magnet (27).

3. Arrangement according to claim 1, **characterized in that** the secondary magnet (27) is arranged in an inner capsule (40), which is located in an outer capsule (39) whose dimensions are slightly larger than the dimensions of the inner capsule (40).

4. Arrangement according to claim 1, **characterized in that** the secondary magnet (27) is arranged in an inner capsule (40), which is located in an outer capsule (39) whose dimensions are considerably larger than the dimensions of the inner capsule (40).

5. Arrangement according to claim 3 or 4, **characterized in that** at least one compensation body (33) for avoiding mechanical imbalances is assigned to the secondary magnet (27).

6. Arrangement according to claim 5, **characterized in that** the compensation body (33) consists of a magnetic or magnetizable material.

7. Arrangement according to claim 1, **characterized in that** transmission means (37) are provided which transmit the movement impulses of the secondary magnet (27) via the supporting structure (28, 29, 36) outside of the housing (30).

8. Arrangement according to claim 7, **characterized in that** the at least one magnetic actuator (42, 43) is designed as two permanent magnets (42).

9. Arrangement according to claim 1, **characterized in that** the at least one magnetic actuator (42, 43) is designed as two energized coils (43).

10. Arrangement according to claim 1 or 7, **characterized in that** energy sources are present in the housing (30).

11. Arrangement according to claims 9 and 10, **characterized in that** the energy sources are inductive electricity generation means (44) for the energized coils (43).

12. Arrangement according to claim 11, **characterized in that** the inductive electricity generation means (44) is assigned to the secondary magnet (27) as actuator.

13. Arrangement according to claim 12, **characterized in that** the voltages activated by the inductive electricity generation means (44) are applicable directly or via a control and communication unit (46) as electrical impulses by means of electrodes.

14. Arrangement according to claim 10 and 13, **characterized in that** electrodes (47, 481, 482, 49) for emitting the electrical impulses are provided on the outside of the housing (30).

15. Arrangement according to claim 1 and 7, **characterized in that** the supporting structure (28, 29, 36) is one with the housing (30).

16. Arrangement according to claim 15, **characterized in that** the secondary magnets (271, 272, 273, 274) are fixedly connected to the housing (30).

## Revendications

1. Dispositif de génération sans contact d'impulsions mécaniques, électriques et magnétiques définies, le dispositif ayant au moins un aimant primaire (20) qui peut être déplacé par un premier actionneur (21, 22, 23, 24), au moins un aimant secondaire (27), qui peut être déplacé par l'aimant primaire (20) dans un boîtier (30), étant situé dans le champ magnétique (26) de l'aimant primaire (20), dans lequel
- une structure de support (28, 29, 36) est présente dans le boîtier (30), l'aimant secondaire (27) étant disposé dans la structure de support (28, 29, 36),
- au moins un actionneur magnétique (42, 43) est associé à l'aimant secondaire (27), ledit actionneur magnétique (42, 43) ayant un effet déviant sur l'aimant secondaire (27) et influençant un champ magnétique résultant d'aimants primaires (20) et aimants secondaires (27), de telle façon que des impulsions de mouvement de l'aimant secondaire (27) sont activées.

2. Dispositif selon la revendication 1, **caractérisé en ce que** l'aimant secondaire (27) est monté de manière mobile de tous les côtés au moyen d'un milieu (28) à faible viscosité dans une capsule (29) dont les dimensions internes sont visiblement plus grandes que les dimensions de l'aimant secondaire (27).

3. Dispositif selon la revendication 1, **caractérisé en ce que** l'aimant secondaire (27) est disposé dans une capsule interne (40) qui est située dans une capsule externe (39) dont les dimensions sont légèrement plus grandes que les dimensions de la capsule interne (40).

4. Dispositif selon la revendication 1, **caractérisé en ce que** l'aimant secondaire (27) est disposé dans une capsule interne (40) qui est située dans une capsule externe (39) dont les dimensions sont nettement plus grandes que les dimensions de la capsule interne (40).

5. Dispositif selon la revendication 3 ou 4, **caractérisé en ce qu'**au moins un corps de compensation (33) pour éviter des déséquilibres mécaniques est associé à l'aimant secondaire (27).

6. Dispositif selon la revendication 5, **caractérisé en ce que** le corps de compensation (33) est constitué d'un matériau magnétique ou magnétisable.

7. Dispositif selon la revendication 1, **caractérisé en ce que** des moyens de transmission (37) sont prévus qui transmettent les impulsions de mouvement de l'aimant secondaire (27) par la structure de support (28, 29, 36) à l'extérieur du boîtier (30).

8. Dispositif selon la revendication 7, **caractérisé en ce que** l'au moins un actionneur magnétique (42, 43) est réalisé sous la forme de deux aimants permanents (42).

9. Dispositif selon la revendication 1, **caractérisé en ce que** l'au moins un actionneur magnétique (42, 43) est réalisé sous la forme de deux bobines (43) traversées par un courant.

10. Dispositif selon la revendication 1 ou 7, **caractérisé en ce que** des sources d'énergie sont présentes dans le boîtier (30).

11. Dispositif selon les revendications 9 et 10, **caractérisé en ce que** les sources d'énergie sont des moyens de production inductive d'électricité (44) pour les bobines (43) traversées par un courant.

12. Dispositif selon la revendication 11, **caractérisé en ce que** le moyen de production inductive d'électricité (44) est associé à l'aimant secondaire (27) comme actionneur.

13. Dispositif selon la revendication 12, **caractérisé en ce que** les voltages activés par les moyens de production inductive d'électricité (44) sont applicables directement ou par une unité de contrôle et de communication (46) en tant qu'impulsions électriques au moyen d'électrodes.

14. Dispositif selon la revendication 10 et 13, **caractérisé en ce que** des électrodes (47, 481, 482, 49) pour émettre les impulsions électriques sont prévues à l'extérieur du boîtier (30).

15. Dispositif selon la revendication 1 et 7, **caractérisé en ce que** la structure de support (28, 29, 36) fait un avec le boîtier (30).

16. Dispositif selon la revendication 15, **caractérisé en ce que** les aimants secondaires (271, 272, 273, 274) sont liés au boîtier (30) de manière fixe.
